# EUROPEAN PATENT APPLICATION

(11) **EP 4 790 143 A2**
(43) Date of publication of application: **12.08.2026**
(21) Application number: 26157362.0
(22) Date of filing: 09.02.2026
(51) Int. Cl.: C12Q 1/26, C12Q 1/48, G01N 33/52

(54) **DRY PAPER-BASED TEST STRIP POTASSIUM ASSAY**

(30) Priority: 07.02.2025 US 202563755697 P
(71) Applicant: Jana Care, Inc., Watertown, MA 02472 (US)
(72) Inventor: Williams, Richard E., Watertown, 02472 (US); Shah, Nishal N., Watertown, 02472 (US); Chan, Brendan C., Watertown, 02472 (US); Prokopis, Mitchell R., Watertown, 02472 (US); Grover, Kanishka, Watertown, 02472 (US)
(74) Representative: Marks & Clerk LLP

(57) **Abstract**

A device may include a filter layer adapted to separate a drop of plasma from components of a drop of whole blood from a single fingerstick. A device may include an interference mitigation composition in a configuration where it contacts the whole blood and/or the drop of plasma to deplete or reduce endogenous substances therein. A device may include an optical analysis layer comprising an enzymatic cascade detection system and adapted to receive the drop of plasma from the filter layer, wherein the optical analysis layer is accessible to spectroscopic interrogation, wherein the enzymatic cascade detection system includes phosphoenolpyruvate or a phosphoenolpyruvate salt, a pyruvate kinase that converts the phosphoenolpyruvate into pyruvate, and an enzyme readout system that generates a detectable chromophore from the pyruvate, wherein potassium concentration within the drop of plasma can be extracted from a rate of generation of the detectable chromophore from the enzymatic cascade system.

## Description

### CROSS-REFERENCE TO RELATED APPLICATION

This application relates to, incorporates by reference for all purposes, and claims priority to United States Application Serial Number 63/755,697 filed on February 7, 2025.

### BACKGROUND

Healthy levels of potassium in plasma ranges from 3.7 mM to 5.2 mM. Abnormal potassium levels can indicate impaired kidney function and can be a side effect of drugs used to treat chronic kidney disease and heart failure. A need exists for an easy-to-use method for patients and healthcare workers to check potassium levels in whole blood.

### SUMMARY

In some aspects, the techniques described herein relate to a test strip including a layered structure including a top layer, a bottom layer, and active layers positioned between the top and bottom layers, the active layers including: a filter layer adapted to separate a drop of plasma from components of a drop of whole blood from a single fingerstick; an interference mitigation composition positioned between the top layer and the bottom layer in a configuration where it contacts the whole blood and/or the drop of plasma to deplete or reduce endogenous substances therein; an optical analysis layer including an enzymatic cascade detection system and adapted to receive the drop of plasma from the filter layer, wherein the optical analysis layer is accessible to spectroscopic interrogation, wherein the enzymatic cascade detection system includes phosphoenolpyruvate or a phosphoenolpyruvate salt, a pyruvate kinase that converts the phosphoenolpyruvate into pyruvate, and an enzyme readout system that generates a detectable chromophore from the pyruvate, wherein the pyruvate kinase is a first stage of an enzymatic cascade, wherein potassium concentration within the drop of plasma can be extracted from a rate of generation of the detectable chromophore from the enzymatic cascade detection system, wherein the detectable chromophore has an absorbance peak that does not overlap with any oxyhemoglobin absorbance peak.

In some aspects, the techniques described herein relate to an at-home testing device with replaceable test strip, the device including: a light source; a multi-channel detector; a test strip insertion port adapted to receive the test strip; and the test strip of any one of the preceding claims, wherein the light source is adapted to illuminate the optical analysis layer with visible light, wherein the multi-channel detector is adapted to detect reflected light from the optical analysis layer.

In some aspects, the techniques described herein relate to a method including: (a) receiving a drop of whole blood from a single fingerstick; (b) filtering the drop of whole blood to separate a drop of plasma from other cellular blood components; (c) exposing the drop of plasma to an enzymatic cascade detection system; (d) measuring a rate of generation of chromophore from the enzymatic cascade detection system; (e) applying a hemolysis correction algorithm to account for intracellular potassium; and (f) determining a potassium concentration in the drop of whole blood using the rate of generation of chromophore and following application of the hemolysis correction algorithm.

### BRIEF DESCRIPTION OF THE DRAWINGS AND APPENDIX

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention.
Fig. 1 includes an overview of the system for detecting potassium using a dry chemistry test strip, a detector, and a result output.
Fig. 2 shows the reaction scheme for turning phosphoenolpyruvate (PEP) into a chromophore and the mechanism of the enzymatic cascade to detect a chromophore as a function of potassium concentration.
Fig. 3 depicts a schematic view of correlating reaction rate from absorbance to potassium concentration and determination of initial rate of reaction, highlighting the linear region of the dye signal as a function of concentration, as depicted by the thicker portion beginning at the origin of each trace.
Fig. 4 depicts determination of the relationship between potassium concentration and reaction rate in solution.
Fig. 5A depicts a schematic view of correlating reaction rate from absorbance to potassium concentration and determination of the relationship of reaction rate to K⁺ concentration. Figure 5B indicates predictive value of reaction rate.
Fig. 6 includes a schematic of the strip assembly.
Fig. 7 shows absorption spectra for the detector including multi-channel monitoring at 30 nm intervals.
Fig. 8A depicts UV-Vis absorption spectra of reaction color and hemolysis detection for hemoglobin and oxyhemoglobin using 2 channels.
Fig. 8B highlights peak detection of reaction color and hemolysis detection using 2 of 8 available detector channels for measuring hemoglobin and chromophore.
Fig. 9A describes the relationship of percentage of hemolysis to concentration of K⁺ₕₑₘₒ.
Fig. 9B describes the relationship of percentage of hemolysis to absorbance at 415 nm (F1). Inset depicts concentration of K⁺ₕₑₘₒ as a function of absorbance.
Fig. 9C describes the relationship of time to absorbance at 415 nm (F1) and 630 nm (F7).
Fig. 9D describes the relationship of reaction rate measured in AU/min to the total concentration of K⁺ and determination of potassium in plasma **[K⁺ₚₗₐₛₘₐ]** after correcting for **[K⁺ₕₑₘₒ]**.
Fig. 10A shows a comparison of the efficacy of *Mn*²⁺ and *Mg*²⁺ as the divalent cation. For each pair of dots at a given potassium concentration, the upper dot relates to manganese and the lower dot relates to magnesium.
Fig. 10B depicts a comparison of various combinations of *Mn*²⁺ and *Mg*²⁺ to confirm higher molar efficiency of *Mn*²*⁺.* For each paid of bars, the left bar relates to 1mM potassium and the right bar relates to 10mM potassium.
Fig. 11A shows the effect of temperature, time, and stabilizers on reaction rate.
Fig. 11B shows the effect of drying time and temperature on reaction rate with added stabilizers (BST).
Fig. 12 shows the effect of the addition of polyethylene glycol on reaction rate.
Fig. 13 shows the performance of the test strip when detecting potassium in buffer solution. The graph depicts the measured potassium concentration in test strips versus expected potassium concentrations defined using NIST standards.
Fig. 14 illustrates a comparative evaluation of two optical analysis methods-traditional absorbance (Abs) and the Kubelka-Munk (K-M) transformation-applied to reflectance-based measurements.
Fig. 15 illustrates the effect of manganese ion (Mn²⁺) concentration on the absorbance response of an ADP-dependent colorimetric assay.
Fig. 16 illustrates the effect of manganese ion (Mn²⁺) concentration on the absorbance response of an ADP-dependent colorimetric assay.
Fig. 17A, 17B, and 17C illustrate the effect of polyethylene glycol (PEG) on reaction rate enhancement in a potassium-dependent enzymatic assay conducted at a manganese ion (Mn²⁺) concentration of 20 mM. The figure presents three kinetic plots corresponding to different pyruvate kinase (PK) enzyme concentrations (4 U/mL, 2 U/mL, and 1 U/mL). Each plot shows time-dependent absorbance curves for multiple potassium ion (K⁺) concentrations. The figure demonstrates that the presence of PEG significantly increases reaction rates and improves resolution among the K⁺-dependent response curves.

### DETAILED DESCRIPTION

Before the present disclosure is described in further detail, it is to be understood that the disclosure is not limited to the embodiments described. It is also understood that the terminology used herein is for the purpose of describing particular embodiments only and is not intended to be limiting. As used herein, the singular forms "a", "an", and "the" include plural embodiments unless the context clearly dictates otherwise.

It should be apparent to those skilled in the art that many additional modifications beside those already described are possible without departing from the inventive concepts. In interpreting this disclosure, all terms should be interpreted in the broadest possible manner consistent with the context. Variations of the term "comprising" or "including" should be interpreted as referring to elements, components, or steps in a non-exclusive manner, so the referenced elements, components, or steps may be combined with other elements, components, or steps that are not expressly referenced. When two or more ranges for a particular value are recited, this disclosure contemplates all combinations of the upper and lower bounds of those ranges that are not explicitly recited. For example, recitation of a value of between 1 and 10 or between 2 and 9 also contemplates a value of between 1 and 9 or between 2 and 10. All ranges are inclusive of the upper and lower value.

Healthy potassium levels range from around 3.7 mM to 5.2 mM in plasma in humans. Hyperkalemia, or high levels of potassium above 6 mM, may be an indicated of impaired kidney function and can cause heart problems including fatal arrythmias. Hyperkalemia is a known side effect of drugs used to treat chronic kidney disease and heart failure. There is a need for the ability to accurately test potassium levels in a repeatable, accessible manner for many patients.

Traditional finger stick methods can cause hemolysis, or the breakdown of red blood cells. Hemolysis often releases excess potassium into blood plasma, which can cause potassium levels to appear abnormally high in laboratory testing. By introducing an enzymatic cascade into the test strips, these levels can be more accurately measured. In the enzymatic cascade, phosphoenolpyruvate undergoes a reaction catalyzed by pyruvate kinase to transform into pyruvate. The pyruvate is then oxidized in two further steps to produce a chromophore in a 1:1 phosphoenolpyruvate:chromophore ratio.

In this reaction, potassium affects the rate of the reaction but not the end point. As phosphoenolpyruvate is converted into the chromophore, the absorbance of light at a particular wavelength by the product increases in a linear proportion to the chromophore concentration. Since potassium affects the rate of the reaction, the initial, linear rate of reaction is important before the rate slows down due to substrate depletion. The rate of reaction is dependent on the concentration of potassium, with higher concentrations of potassium reacting faster before a rate decrease due to substrate depletion.

This results in differing concentrations of potassium correlating to differing rates of reaction, with the rate of reaction being linearly proportional to the concentration of K⁺. The rate of reaction can then be correlated back to potassium concentration to determine the sensitivity and accuracy of the test.

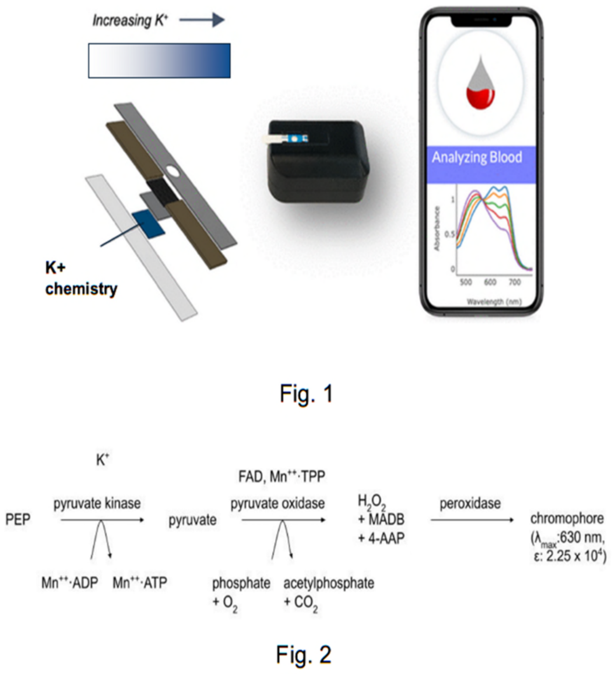

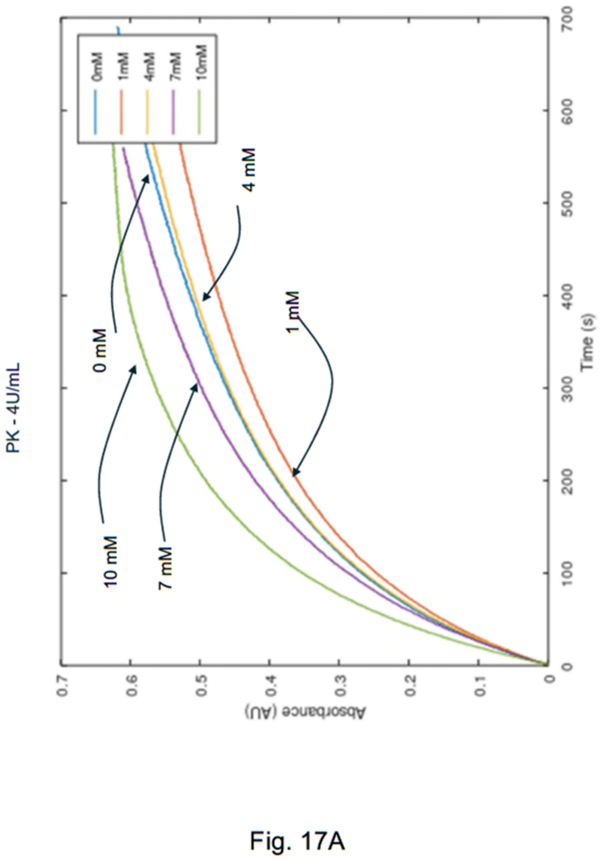

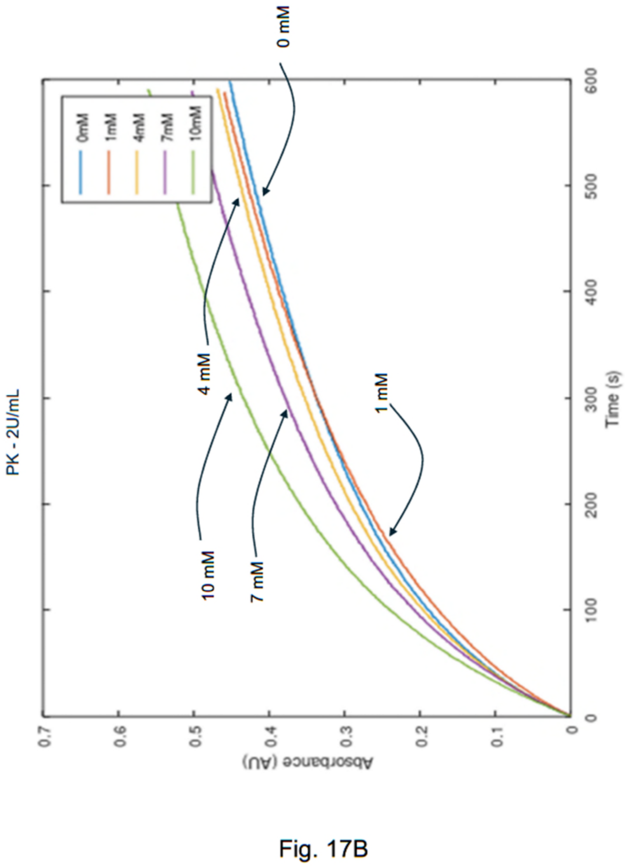

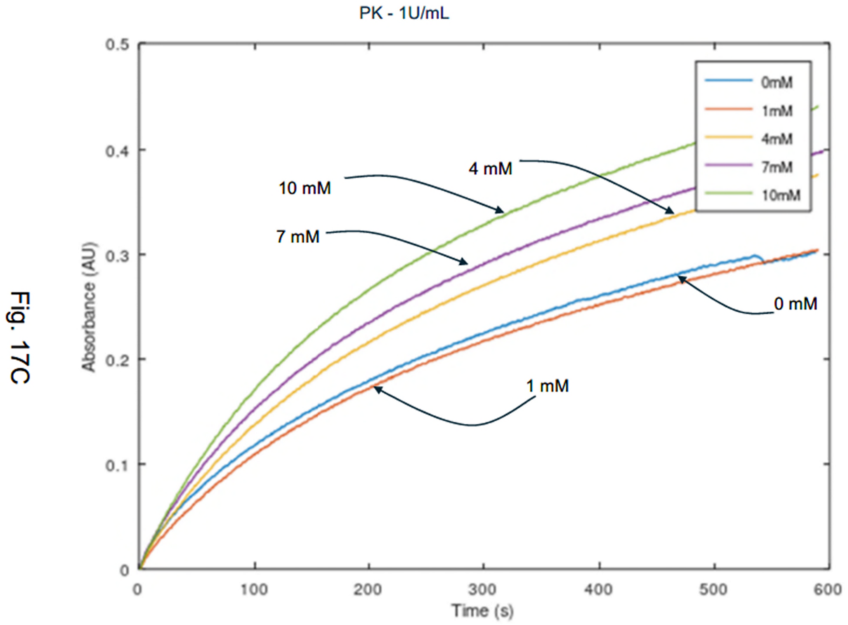

Samples are deposited onto test strips and the potassium concentrate is read out as shown in Fig. 1. The addition of bovine serum albumin (BSA), sucrose, and trehalose improve the preservation of enzyme activity after drying onto the paper test strips, proving the importance of stabilizers on the test strips. Addition of polyethylene glycol (PEG) to the test strips both increases potassium sensitivity and increases the rate of reaction, possibly due to an increase in dried enzyme stability. Referring to Fig. 17A, 17B, and 17C, the addition of polyethylene glycol (PEG) leads to substantial improvements in reaction kinetics within the colorimetric assay system. Fig. 17A, 17B, and 17C depict measurements obtained at a fixed Mn²⁺ concentration of 20 mM, while varying the concentration of pyruvate kinase (PK). The data in Fig. 17A, 17B, and 17C correspond to PK levels of 4 U/mL, 2 U/mL, and 1 U/mL, respectively. In each case, absorbance is plotted as a function of time for several K⁺ concentrations.

Across all PK concentrations, the inclusion of PEG produces higher overall absorbance values and faster initial reaction rates and improved differentiation among the K⁺-dependent signals. At each PK concentration, the curves corresponding to different K⁺ levels display greater separation when PEG is present.

The data collectively show that PEG acts as a performance-enhancing reagent within the assay mixture, increasing signal amplitude by approximately three-fold and improving the discriminatory power of the assay with respect to K⁺ concentration. The combination of elevated Mn²⁺ (20 mM) and PEG yields a more robust and sensitive reaction system than previously available formulations, enabling clearer kinetic resolution than comparative assays such as POT83.

In one aspect, a test strip contains a layered structure with a top layer, a bottom layer, and active layers positioned between the top and bottom layers. The active layers include a filter layer adapted to separate a drop of plasma from components of a drop of whole blood from a single fingerstick, an interference mitigation composition positioned between the top layer and the bottom layer in a configuration where it contacts the whole blood and/or the drop of plasma to deplete or reduce endogenous substances therein, and an optical analysis layer comprising an enzymatic cascade detection system and adapted to receive the drop of plasma from the filter layer. The optical analysis layer is accessible to spectroscopic interrogation. The enzymatic cascade detection system includes phosphoenolpyruvate or a phosphoenolpyruvate salt, a pyruvate kinase that converts the phosphoenolpyruvate into pyruvate, and an enzyme readout system that generates a detectable chromophore from the pyruvate. The pyruvate kinase is the first stage in an enzymatic cascade, wherein potassium concentration within the drop of plasma is extracted from a rate of generation of the detectable chromophore from the enzymatic cascade detection system. The detectable chromophore has an absorbance peak that does not overlap with any oxyhemoglobin absorbance peak.

In some cases, the enzymatic cascade detection system includes co-reactants understood to be relevant to the function of the system. In some cases, the enzymatic cascade detection system includes adenosine diphosphate. In some cases, the enzymatic cascade detection system includes thiamin pyrophosphate. In some cases, the enzymatic cascade detection system includes flavin adenine dinucleotide. In some cases, the co-reactants may be divalent cations, such as Mg²⁺, Ca²⁺, Zn²⁺, Ba²⁺, Ni²⁺, Co²⁺, Cr²⁺, Cu²⁺, or a combination thereof. The co-reactants may also be an inorganic phosphate source such as sodium phosphate buffer or lithium phosphate.

The pyruvate kinase may be from a eukaryotic species. The pyruvate kinase may be mammalian. The pyruvate kinase may be from a prokaryotic species (e.g., bacterial pyruvate kinase with glutamate exhibits the necessary potassium dependence). The test strip may be substantially free of sodium ions. The phosphoenolpyruvate salt may be not a sodium salt. The test strip may be substantially free of potassium ions. The phosphoenolpyruvate may be not a potassium salt. The phosphoenolpyruvate salt may be a phosphoenolpyruvate salt that is neither a potassium nor a sodium salt (e.g., other alkali cations or cyclohexylammonium salts) or a combination thereof.

The enzyme readout system may comprise a pyruvate oxidase, a peroxidase, and a Trinder pair. The Trinder pair may comprise N,N-Bis(4-sulfobutyl)-3,5-dimethylaniline, disodium salt (MADB) and 4-aminoantipyrine. Without wishing to be bound by any particular theory, it is believed that substantially all known Trinder pairs have absorption peaks within a window that allows suitable function of the present invention, while allowing detection of hemoglobin necessary for the hemolysis correction algorithm. Other viable Trinder pair alternatives include ADPS, ADOS, ALPS, DAOS, HDAOS, MAOS, TODB, TOPS, and TOOS. Table 1 presents data for example Trinder reagents including specific absorption maxima and corresponding molar extinction coefficients.

**Table 1**

| Trinder Reagent | Product Number | Absorption Peak | Extinction Coefficient |
|---|---|---|---|
| ADOS | (OC01) | 542 | 2.72 E4 |
| ADPS | (OC02) | 540 | 2.79E4 |
| DAOS | (OC06) | 593 | 1.75E4 |
| HDAOS | (OC08) | 583 | 1.73E4 |
| MADB | (OC21) | 630 | 1.65E4 |
| MAOS | (OC11) | 630 | 2.25E4 |
| TOOS | (OC13) | 555 | 3.92 E4 |
| TOPS | (OC14) | 550 | 3.74E4 |
| TODB | (OC22) | 550 | 3.8E4 |
| ALPS | (OC04) | 561 | 4.13E4 |

The test strip can include various buffers, such as MES buffers (2-(N-morpholino)ethanesulfionic acid), phosphate buffers, or a combination thereof.

The test strip may be substantially free of ammonium ions. The test strip may be substantially free of calcium ions. The interference mitigation composition may be adapted to reduce or eliminate sodium ions. The interference mitigation composition may comprise a coronate or a cryptate to reduce or eliminate the sodium ions. The interference mitigation composition may comprise the coronate. The coronate may be formed from ion-crown ether complexing. The cryptate may be formed from ion-cryptand complexing.

The interference mitigation composition may be adapted to reduce or eliminate calcium ions. The interference mitigation composition may comprise a coronate or a cryptate to reduce or eliminate the calcium ions. The interference mitigation composition may comprise a crown ether, a cryptand, a chelating agent (e.g., a citrate, a gluconate, or EDTA), or a combination thereof to reduce or eliminate calcium ions.

The interference mitigation composition may be adapted to reduce or eliminate ammonium ions. The interference mitigation composition may comprise a crown ether, a cryptand, a chelating agent (e.g., a citrate, a gluconate, or EDTA), and enzymatic catalyst (e.g., glutamate dehydrogenase) or a combination thereof to reduce or eliminate the ammonium ions.

The optical analysis layer may be a paper layer. The active layers may be paper layers. The active layers may be dried (e.g., heat dried or lyophilized). The test strip may maintain measurement accuracy for up to 1 year stored between 2 °C and 40 °C. The test strip may further comprise a sample port which is adapted to receive the drop of whole blood. The test strip may further comprise a foam spacer to provide physical spacing between the top layer and the bottom layer.

One exemplary formulation consistent with aspects of this disclosure is described in Table 2.

**Table 2**

| **Component** | **Final Concentration** |
|---|---|
| MES buffer (pH 6 - 7) | 10 - 200 mM |
| Phosphate Buffer | 5 - 100 mM |
| MnCl₂ | 1 - 20 mM |
| Sucrose | 0.1% - 10% (w/v) |
| Trehalose | 0.1% - 10% (w/v) |
| Bovine Serum Albumin (BSA) | 0.05% - 5% (w/v) |
| Polyethylene Glycol (PEG) (MW 200-2000 g/mol) | 0.1% - 10% (w/v) |
| Thiamin pyrophosphate (TPP) | 0.1 - 10 mM |
| Flavin adenine dinucleotide (FAD) | 0.01 - 2 mM |
| 4-aminoantiypyrine (4-AAP) | 0.5 - 20 mM |
| MADB | 0.5 - 20 mM |
| Horseradish peroxidase | 10 - 200 U/mL |
| Pyruvate oxidase | 0.5 - 50 U/mL |
| Pyruvate kinase | 0.5 - 50 U/mL |
| Phosphoenolpyruvate (PEP) | 0.5 - 20 mM |
| Adenosine diphosphate (ADP) | 0.5 - 20 mM |

In some aspects, the test strips are stable for at least 6 months in temperatures ranging from 4 °C to 40 °C with controlled humidity. Humidity may be at least partially controlled with a desiccant. Performance of the strips upon exposure to environmental conditions should be consistent between temperatures of 15 °C to 40 °C and relative humidity between 10% and 80%. The stability may be extended up to 18 months under 15 °C to 40 °C and relative humidity between 10% and 80%. The increased stability may be a result of the addition of stabilizers such as sucrose, trehalose, PEG, and BSA, as well as drying conditions and packaging techniques.

An at home testing device with a test strip contains a light source, a multi-channel detector, a test strip insertion port adapted to receive the test strip, and a test strip described above. The light source is adapted to illuminate the optical analysis layer with visible light, wherein the multi-channel detector is adapted to detect reflected light from the optical analysis layer at: 1) a first wavelength corresponding to an absorbance peak of hemoglobin and 2) a second wavelength corresponding to an absorbance peak of the chromophore. The multi-channel detector is adapted to detect light from the optical analysis layer at the first wavelength and the second wavelength. The multi-channel detector may measure the first wavelength and the second wavelength simultaneously.

Detection of the first wavelength and the second wavelength may be achieved by a specialized device having a multi-detection system with specialized hardware or a general device running specialized software. While devices on the market may be capable of measuring at multiple wavelengths in the visible spectrum, the inventors are not aware of devices that utilize two wavelengths in the fashion described herein. The wavelength window for the first wavelength (hemoglobin) can be 400 - 420 nm and the window for the second wavelength (chromophore) can be 610 - 630 nm. However, the detector can detect light in multiple channels so that hemoglobin could also be detected in the following additional windows: 430 - 450 nm, 460 - 480 nm, 500 - 520 nm, 540 - 560 nm; and the chromophore could also be detected in the following additional windows: 573 - 593 nm, 660 - 680 nm.

A method includes steps a) through f). Step a) includes receiving a drop of whole blood from a single fingerstick. It will be appreciated that step a) provides for the receiving of a previously obtained drop of whole blood from a single fingerstick and not the step of obtaining the blood from a patient, in the same way as the other aspects of the present invention do not require the step of obtaining the blood from a patient. Step b) includes filtering the drop of whole blood to separate a drop of plasma from other cellular components. Step c) includes exposing the drop of plasma to an enzymatic cascade detection system. Step d) includes measuring a rate of generation of chromophore from the enzymatic cascade detection system. Step e) includes applying a hemolysis correction algorithm to account for intracellular potassium. Step f) includes determining a potassium concentration in the drop of whole blood using the rate of generation of chromophore and following application of the hemolysis correction algorithm. Prior to step c), the blood sample and/or the drop of plasma may be depleted of sodium, ammonium, and/or calcium.

The drop of whole blood may have a volume between 20 microliters and 30 microliters.

The hemolysis correction algorithm may compare absorption between a first wavelength of 410 nm and a second wavelength of 620 nm. The first wavelength and the second wavelength may be measured simultaneously. In some cases, the hemolysis correction algorithm can compare absorption between a first wavelength and a second wavelength, wherein the first wavelength is between 400 nm and 420 nm, between 430 nm and 450 nm, between 460 nm and 480 nm, between 500 nm and 520 nm, or between 540 nm and 560 nm, wherein the second wavelength is between 573 nm and 593 nm, between 610 nm and 630 nm, or between 660 nm and 680 nm

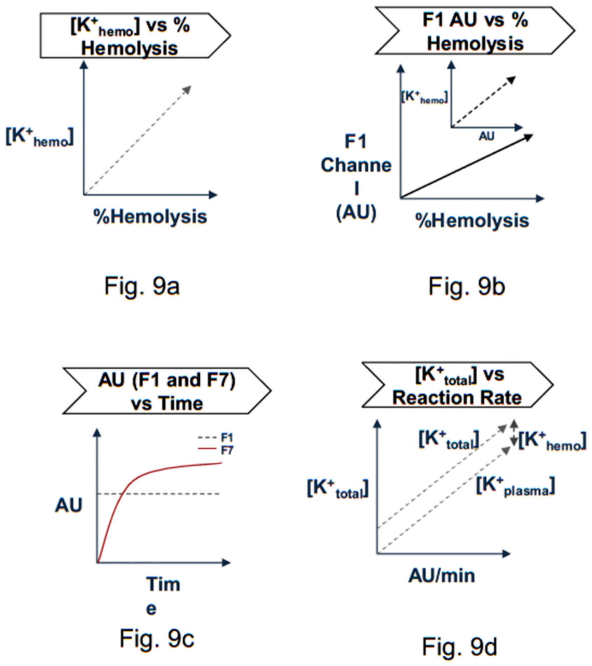

The hemolysis correction algorithm is described below in greater detail with respect to Fig. 9. The hemolysis correction algorithm may include the following steps: 1) determine relationship between % hemolysis and released K⁺ in depleted plasma (K⁺ₕₑₘₒ); 2) determine relationship between % hemolysis and signal measured at 410 nm; 3) determine relationship between the signal measured at 410 nm and K⁺ₕₑₘₒ in K+-depleted plasma from steps 1) and 2); 4) determine relationship between signal at 620 nm and total K⁺ₜₒₜₐₗ in depleted plasma; and 5) during measurement, determine [K⁺ₜₒₜₐₗ] from the signal at 620 nm and [K⁺ₕₑₘₒ] from the signal at 410 nm, and determine K⁺ that is not due to hemolysis [K⁺ₚₗₐₛₘₐ] by subtracting [K⁺ₕₑₘₒ] from [K⁺ₜₒₜₐₗ]. It should be appreciated that these wavelength values can be substituted with wavelength values described above with respect to the first and second wavelengths.

Fitting the data during the measuring of step d) may include a weighted measurement using at least a portion of a standard absorbance equation and a Kubelka-Munk absorbance equation. The weighted measurement will be in the form of Signal = X * Abs + Y * Kubelka-Monk where X and Y will be coefficients with values between 1 and 10.

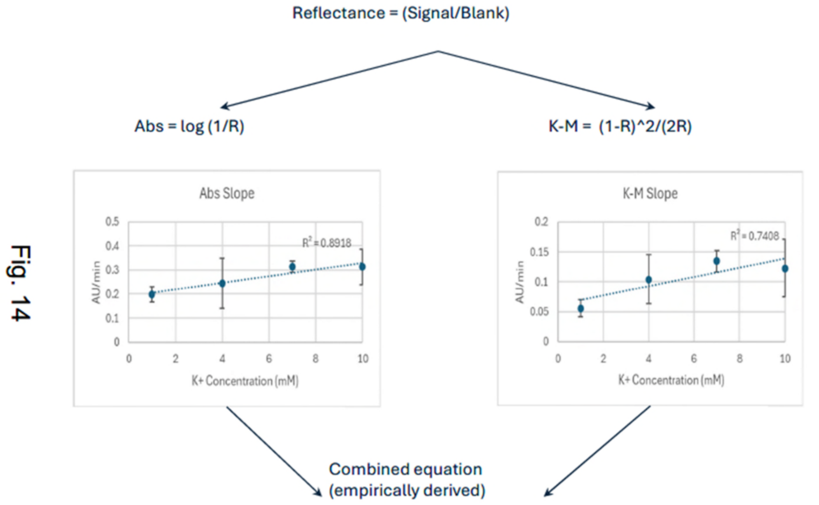

Referring to Fig. 14, a comparison of two analytical approaches used to interpret reflectance-based measurements in colorimetric assays, namely (i) the conventional absorbance transformation and (ii) the Kubelka-Munk (K-M) function. Reflectance (R) is first obtained as the ratio of the detected signal to a blank reference and is transformed according to either method. Under the absorbance model, the absorbance value (Abs) is calculated as the logarithm of the inverse of reflectance (Abs = log(1/R)). Alternatively, the Kubelka-Munk transformation employs a scattering-corrected function defined as K-M = (1 - R)²/(2R), which can be used to linearize colorimetric responses obtained from diffuse reflectance measurements.

The left graph shows the absorbance-based slope obtained by plotting assay rate (AU/min) as a function of potassium ion concentration. The resulting linear fit exhibits a positive correlation. The right graph shows the corresponding K-M-based slope, likewise plotted as assay rate versus potassium concentration, and similarly demonstrates a linear trend. Each plot includes a regression line and associated coefficient of determination (R²), allowing direct comparison of the linearity and sensitivity of the two analytical approaches. The absorbance and Kubelka-Munk transformations may be empirically combined to derive an optimized equation for relating reflectance to analyte concentration.

One possible outcome of the hemolysis correction algorithm is to reject the sample entirely. The sample may be rejected if the measured hemoglobin exceeds a certain threshold value, which might indicate a large amount of hemolysis and consequently a high concentration of intracellular potassium released into the plasma.

The enzyme cascade detection system may comprise manganese ions. A majority of divalent cations within the enzyme cascade detection system may be manganese ions. The enzyme cascade detection system may also be substantially free of manganese ions. The enzymatic cascade detection system may comprise one or more stabilizers selected from the group consisting of bovine serum albumin, sucrose, trehalose, and combinations thereof. The enzyme cascade detection system may comprise polyethylene glycol.

A non-transitory computer readable medium has instructions stored thereon that, when executed by a processor, causes the processor to one or more steps of the methods described herein, including the measuring of step d), the applying of step e), and/or the determining of step f).

The inventors have unexpectedly discovered that their dry, paper-based assays perform significantly better when using manganese as a divalent cation rather than magnesium.

Without wishing to be bound by any particular theory, the inventors have provided inventive solutions to the challenging problem of accurate at-home potassium blood testing. The primary technical hurdles overcome include developing a paper-based dry chemistry approach for the detection of potassium from whole blood for a home setting. By using an enzymatic cascade for chromophore detection, the system can use a combination of specific light sources and detectors to accurately measure absorbed light as a function of potassium concentration. The system also provides an approach for detecting and correcting for hemolysis, a common cause for inflated readings. Additionally, the system may have long-term stability as a function of the dry chemistry and may also include a method for temperature correction.

All references, including publications, patent applications, and patents, cited herein are hereby incorporated by reference to the same extent as if each reference were individually and specifically indicated to be incorporated by reference and were set forth in its entirety herein.

Preferred aspects of this invention are described herein, including the best mode known to the inventors for carrying out the invention. Variations of those preferred aspects may become apparent to those of ordinary skill in the art upon reading the foregoing description. The inventors expect a person having ordinary skill in the art to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

While the invention has been illustrated and described in detail in the foregoing drawings and description, the same is to be considered as illustrative and not restrictive in character, it being understood that only illustrative embodiments thereof have been shown and described and that all changes and modifications that come within the spirit of the invention are desired to be protected. For example, any of the features or functions of any of the embodiments disclosed herein may be incorporated into any of the other embodiments disclosed herein.

The following examples illustrate some embodiments and aspects of the invention. It will be apparent to those skilled in the relevant art that various modifications, additions, substitutions, and the like can be performed without altering the spirit or scope of the invention, and such modifications and variations are encompassed within the scope of the invention as defined in the claims which follow. The following examples do not in any way limit the invention.

### EXAMPLES

### Example 1:

K⁺ concentration in solution and whole blood was measured by monitoring a series of reactions which rely on K⁺ to produce a chromophore with an emission wavelength of 630 nm which are described in Fig. 2. First, phosphoenolpyruvate (PEP) in the presence of pyruvate kinase and K⁺ was converted to pyruvate. Pyruvate was then reacted in the presence of flavin adenine dinucleotide (FAD), phosphate, Mn²⁺ thiamine pyrophosphate (TPP), and pyruvate oxidase followed by peroxidase to produce the chromophore. This set of reactions is a three enzyme cascade with multiple cofactors. Importantly, the stoichiometry of PEP to chromophore is 1:1. K⁺ concentration affects the rate of reaction, but not the endpoint.

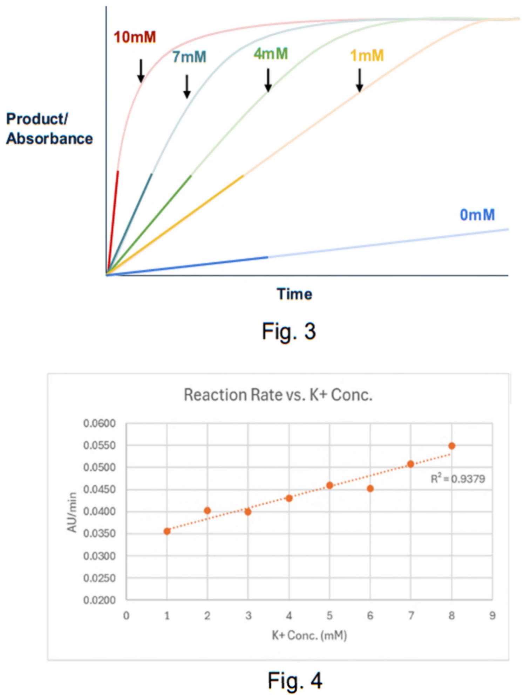

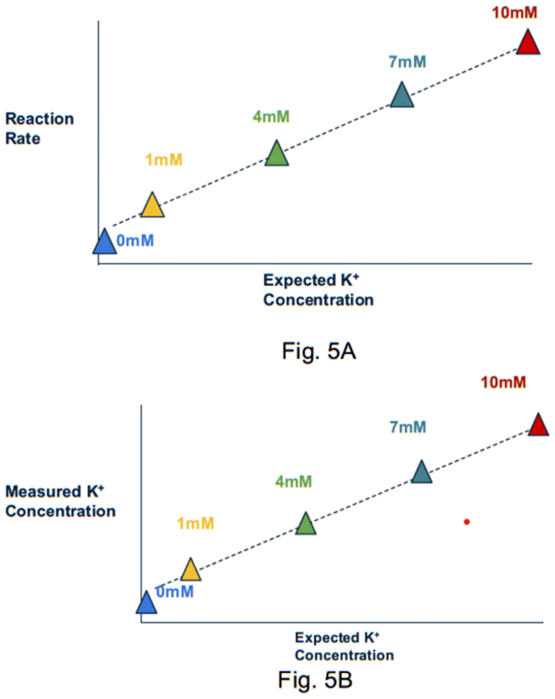

Various concentrations of the K⁺ in the presence of the enzymatic cascade were investigated. As shown in Fig. 3, which depicts the absorbance at 630 nm, as the concentration of K⁺ increases, the faster the enzyme-catalyzed reaction proceeds before slowing down due to substrate depletion. The rate of reaction is the highest of the concentrations tested at 10 mM K⁺, followed by 7 mM, 4 mM, and 1 mM, respectively, as seen in the highlighted portions of the plot in Fig. 3. Different concentrations of K⁺ yielded different rates of reaction. Specifically, concentration of K⁺ value had a linear proportional relationship to the rate of reaction (Fig. 4) by measuring the rate of appearance of the chromophore in solution. The rate of reaction can be correlated back to K⁺ concentration to determine the sensitivity and accuracy of the test as shown in Fig. 5.

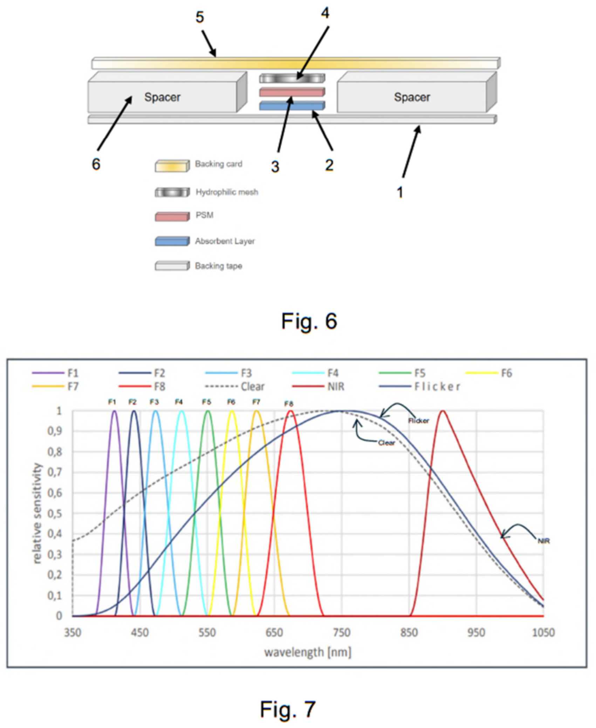

Using the established relationship of the enzymatic cascade in solution, test strips were developed as seen in Fig. 6. Backing tape 1 was selected for the bottom layer to provide an optically clear surface to measure changes in color. CF1, an absorbent layer 2 chosen for minimal volume absorption, highly diffuse reflectance, and general compatibility with the buffer reagents, was placed on top of that as the intended region for the chemistry to occur. Dried enzymes, lyophilized cofactors, dyes and chromophore, and stabilizers were added. The next layer up was the plasma separation membrane (PSM) 3, which filters red blood cells out of whole blood. Dried reaction precursors, lyophilized stabilizers, and ion depleters were added. A layer of hydrophobic mesh 4 was placed on top of the PSM to distribute the sample evenly. Finally, a backing card 5, composed of a polymer such as PVC, PET, or other similar synthetic polymers with a thickness between 0.1 mm and 1.0 mm, to provide structural support was placed on the top. Optional spacers, which may be foam spacers, are depicted.

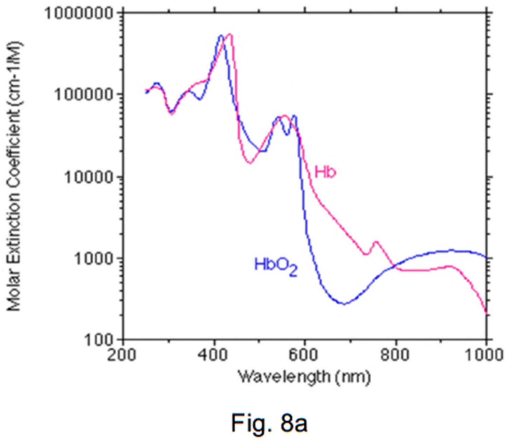

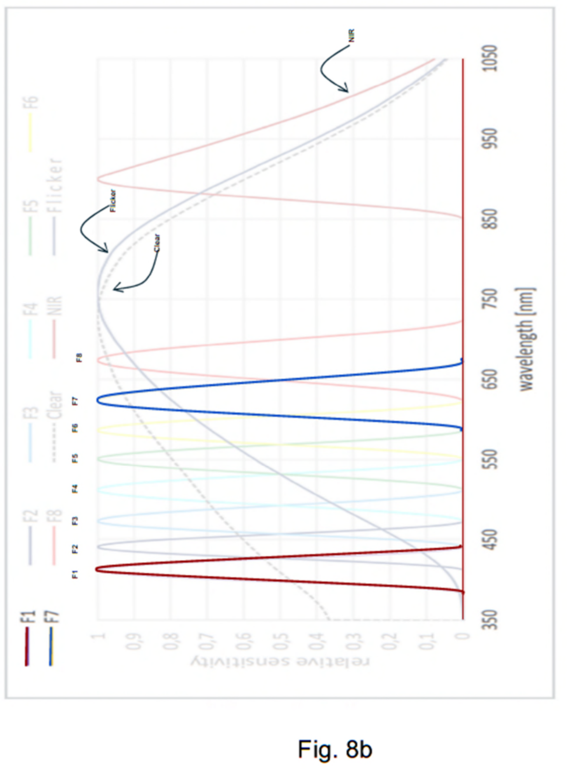

Using Aina C devices, the reaction was measured on paper strips as shown in Fig. 7. F1 through F8 correspond to the 8 channels that were read out at 30 nm intervals. Channels F1 at 415 nm and F7 at 630 were monitored as they correspond to signals given off by hemoglobin and the chromophore, respectively, as shown in Fig. 8A and Fig. 8B. It was important to measure hemoglobin as hemolysis can increase K⁺ concentration, leading to an artificially high reading.

By comparing the AU/min of a range of K⁺ concentration to the AU/min range of hemolysis percentage, the test strips quantitatively determined the amount of K⁺ contributed by hemolysis. Specifically, the relationship between the percentage of hemolysis and the concentration of K⁺ released in depleted plasma ([K⁺ₕₑₘₒ]) was determined (Fig. 9A). Next, the relationship between the absorbance in the F1 channel and the percentage of hemolysis was determined (Fig. 9B). The relationship between the signal in the F1 channel and the K⁺ from depleted plasma in Fig. 9A was determined (Fig. 9C). The relationship between the signal in the F7 channel and the total amount of K⁺ ([K⁺ₜₒₜₐₗ]) was determined (Fig. 9C). During measurements, [K⁺ₜₒₜₐₗ] was determined from channel F7 and [K⁺ₕₑₘₒ] was determined from channel F1. The signal from F1 ([K⁺ₕₑₘₒ]) was then subtracted from the total K⁺ in channel F7 ([K⁺ₜₒₜₐₗ])to give the real K⁺ concentration in plasma ([K⁺ₚₗₐₛₘₐ]) (Fig. 9D).

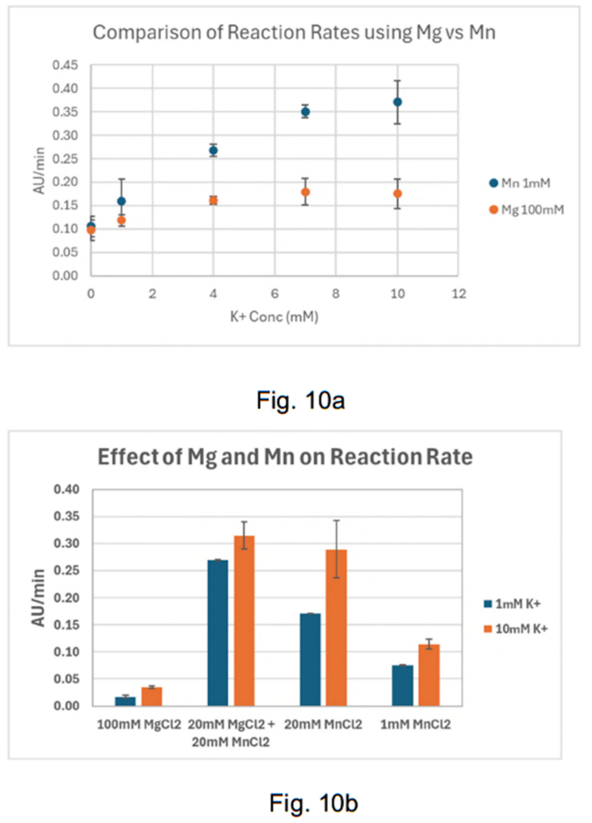

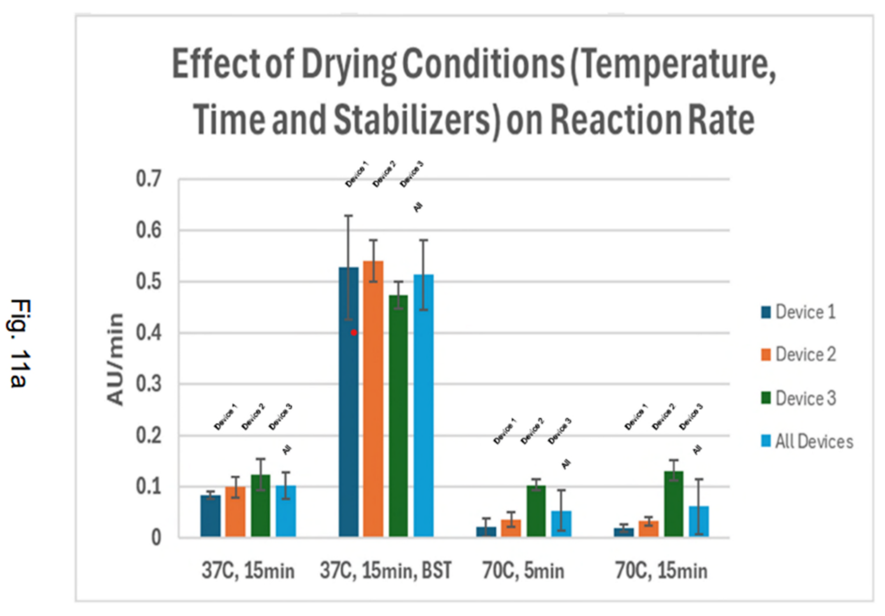

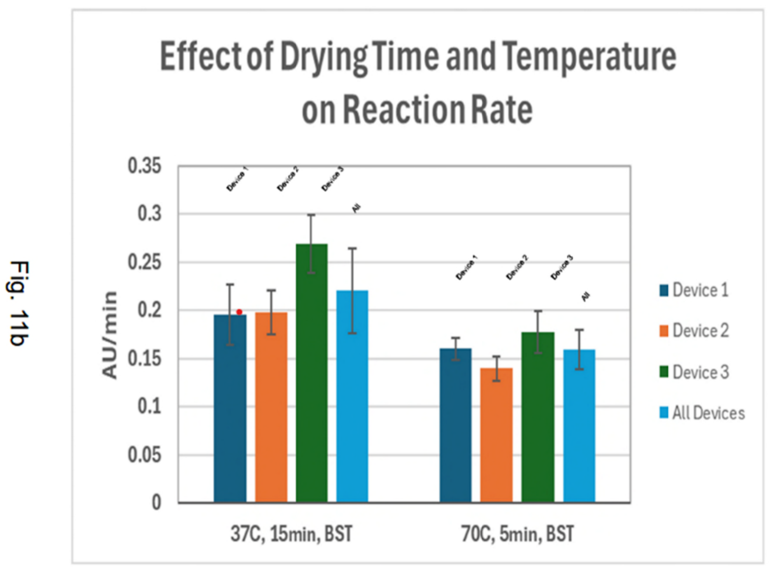

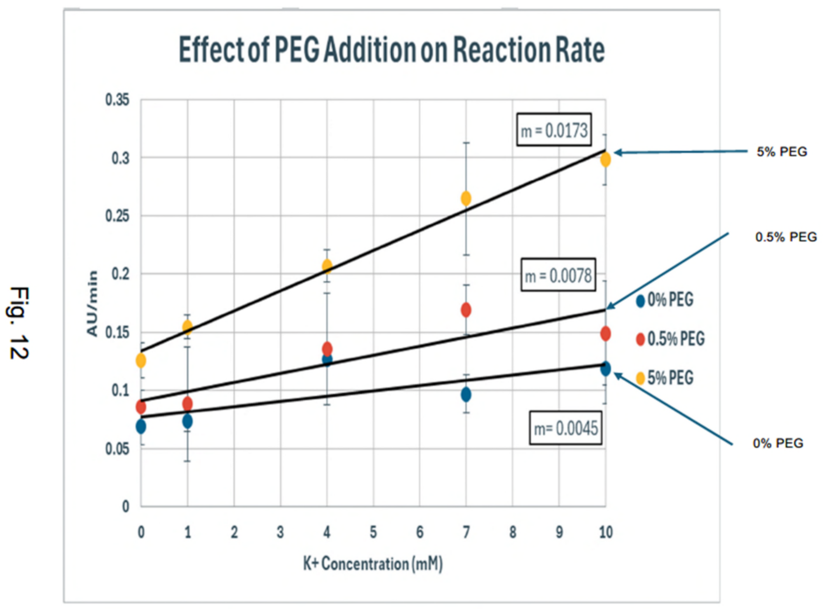

Many factors influencing reaction rates were investigated. Reaction rates using Mg²⁺ were compared to Mn²⁺ and summarized in Fig. 10A and Fig. 10B. Compared to Mg²⁺, addition of Mn²⁺ increases reaction time and generates higher absorbance readings at both 1 mM K⁺ and 10 mM K⁺. Mn²⁺ must be in excess with respect to adenosine diphosphate (ADP) concentration. Increasing the Mn²⁺ concentration increased absorbance three times and allowed for increased resolution of K⁺ concentrations. Fig. 11A and Fig. 11B describe the effect of temperature and the addition of bovine serum albumin (BSA), sucrose, and trehalose on the test strips. BSA, sucrose, and trehalose improve the preservation of enzyme activity after drying onto the paper test strips. The addition of polyethylene glycol (PEG) to the test strips also increases reaction rate (Fig. 12), improving preservation of enzyme activity and potassium sensitivity. Fig. 12 depicts a comparison of different concentrations of PEG-8000 as a stabilizer during drying shows 5% PEG allows for ~4x improvement in enzyme activity over no PEG and >2x improvement over 0.5% PEG.

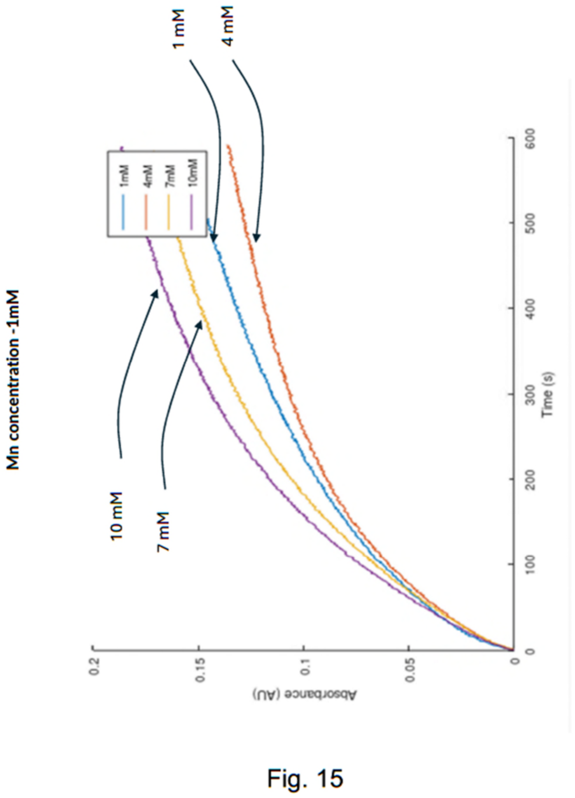

Without wishing to be bound by any particular theory, and with reference to Fig. 15 and Fig, 16, it is believed that maintaining the concentration of manganese ions (Mn²⁺) in excess relative to the concentration of adenosine diphosphate (ADP) achieves robust assay performance. Fig, 15 presents absorbance-versus-time curves collected using an Mn²⁺ concentration of 1 mM. Under these conditions, the overall absorbance increase is modest.

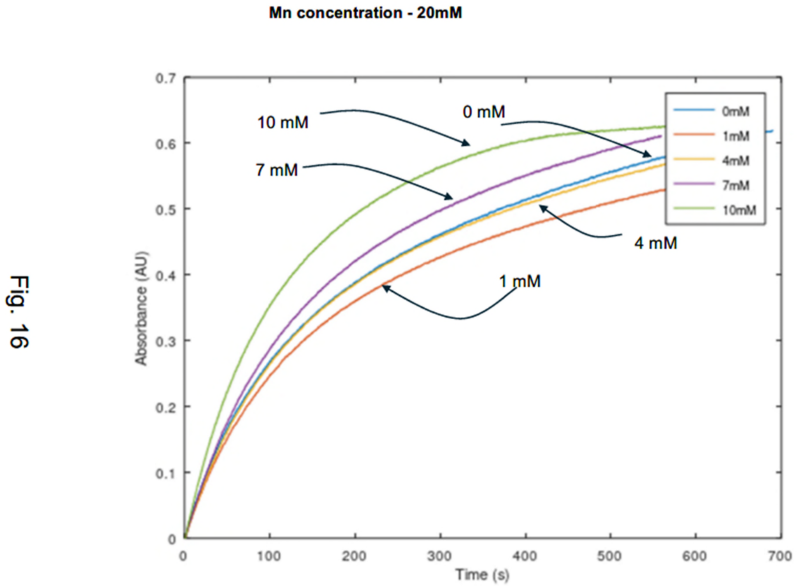

In contrast, Fig. 16 shows corresponding measurements performed with an Mn²⁺ concentration of 20 mM. At this elevated Mn²⁺ level, the absorbance signal increases by approximately three-fold relative to the 1 mM condition. The kinetic profiles display steeper initial slopes and higher overall signal amplitudes.

By adjusting Mn²⁺ concentration to levels such as 20 mM or higher, the assay achieves improved sensitivity to K⁺ concentration, broader dynamic range, and more reliable calibration behavior.

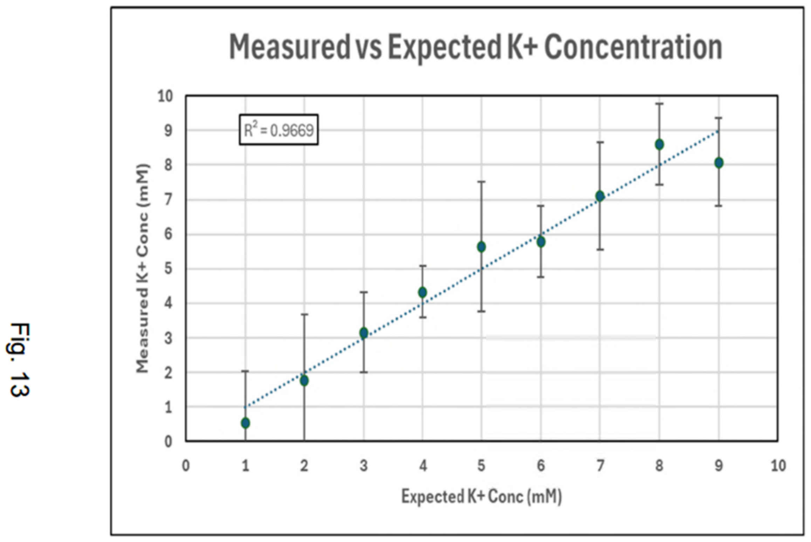

The analytical limit of detection (LoD) of K⁺ in buffer using the enzymatic cascade is 0.97 mM, which is below the clinically relevant range of 2.3 mM to 7 mM. A healthy individual should have a range between 3.7 and 5.2 mM. As shown in Fig. 13, the enzymatic approach on test strips has a relatively high predictive value for potassium detection with an R² of 0.9669.

### EQUIVALENTS AND SCOPE

The recitation of a listing of elements in any definition of a variable herein includes definitions of that variable as any single element or combinations (or subcombinations) of listed elements. The recitation of an embodiment herein includes that embodiment as any single embodiment or in combination with any other embodiments or portions thereof. Those skilled in the art will recognize or be able to ascertain using no more than routine experimentation, many equivalents to the specific embodiments of the invention described herein.

In addition to the features described above and elsewhere herein, the present disclosure also includes the following clauses:
1. A test strip comprising a layered structure comprising a top layer, a bottom layer, and active layers positioned between the top and bottom layers, the active layers including:
   a filter layer adapted to separate a drop of plasma from components of a drop of whole blood from a single fingerstick;
   optionally an interference mitigation composition positioned between the top layer and the bottom layer in a configuration where it contacts the whole blood and/or the drop of plasma to deplete or reduce endogenous substances therein;
   an optical analysis layer comprising an enzymatic cascade detection system and adapted to receive the drop of plasma from the filter layer,
   wherein the optical analysis layer is accessible to spectroscopic interrogation,
   wherein the enzymatic cascade detection system includes phosphoenolpyruvate or a phosphoenolpyruvate salt, a pyruvate kinase that converts the phosphoenolpyruvate into pyruvate, and an enzyme readout system that generates a detectable chromophore from the pyruvate, wherein the pyruvate kinase is a first stage of an enzymatic cascade, wherein
   potassium concentration within the drop of plasma can be extracted from a rate of generation of the detectable chromophore from the enzymatic cascade detection system,
   wherein the detectable chromophore has an absorbance peak that does not overlap with any oxyhemoglobin absorbance peak.
2. The test strip of clause 1, wherein the pyruvate kinase is from a eukaryotic species.
3. The test strip of clause 2, wherein the pyruvate kinase is mammalian.
4. The test strip of clause 1, herein the pyruvate kinase is from a prokaryotic species.
5. The test strip of any one of the preceding clauses, wherein the test strip is substantially free of sodium ions.
6. The test strip of any one of the preceding clauses, wherein the phosphoenolpyruvate salt is not a sodium salt.
7. The test strip of any one of the preceding clauses, wherein the test strip is substantially free of potassium ions.
8. The test strip of any one of the preceding clauses, wherein the phosphoenolpyruvate salt is not a potassium salt.
9. The test strip of any one of the preceding clauses, wherein the phosphoenolpyruvate salt is neither a potassium salt nor a sodium salt.
10. The test strip of any one of the preceding clauses, wherein the test strip is substantially free of ammonium ions.
11. The test strip of any one of the preceding clauses, wherein the test strip is substantially free of calcium ions.
12. The test strip of any one of the preceding clauses, the system comprising the interference mitigation composition.
13. The test strip of the immediately preceding clause, wherein the interference mitigation composition is adapted to reduce or eliminate sodium ions.
14. The test strip of the immediately preceding clause, wherein the interference mitigation composition comprises a coronate or a cryptate to reduce or eliminate the sodium ions.
15. The test strip of clause 14, wherein the interference mitigation composition comprises the coronate.
16. The test strip of the immediately preceding clause, where the coronate is formed from ion-crown ether complexing.
17. The test strip of clause 14, wherein the interference mitigation composition comprises the cryptate.
18. The test strip of the immediately preceding clause, wherein the cryptate is formed from ion-cryptand complexing.
19. The test strip of any one of clauses 12 to the immediately preceding clause, wherein the interference mitigation composition is adapted to reduce or eliminate calcium ions.
20. The test strip of clause 19, wherein the interference mitigation composition comprises a coronate or a cryptate to reduce or eliminate the calcium ions.
21. The test strip of clause 19, wherein the interference mitigation composition comprises a crown ether, a cryptand, a chelating agent (e.g., a citrate, a gluconate, or EDTA), or a combination thereof to reduce or eliminate the calcium ions.
22. The test strip of any one of clauses 12 to the immediately preceding clause, wherein the interference mitigation composition is adapted to reduce or eliminate ammonium ions.
23. The test strip of the immediately preceding clause, wherein the interference mitigation composition comprises a crown ether, a cryptand, a chelating agent (e.g., a citrate, a gluconate, or EDTA), or a combination thereof to reduce or eliminate the ammonium ions.
24. The test strip of any one of the preceding clauses, wherein at least the optical analysis layer is a paper layer.
25. The test strip of any one of the preceding clauses, wherein the active layers are paper layers.
26. The test strip of any one of the preceding clauses, wherein the active layers are dried (e.g., heat dried or lyophilized).
27. The test strip of any one of the preceding clauses, wherein the test strip maintains measurement accuracy for at length of time of at least one year stored at a temperature of between 2 °C and 40 °C.
28. The test strip of any one of the preceding clauses, the test strip further comprising a sample port adapted to receive the drop of whole blood.
29. The test strip of any one of the preceding clauses, the test strip further comprising a foam spacer to provide physical spacing between the top layer and the bottom layer.
30. The test strip of any one of the preceding clauses, wherein the enzyme readout system comprises a pyruvate oxidase, a peroxidase, and a Trinder pair.
31. The test strip of the immediately preceding clause, wherein the Trinder pair comprises N,N-Bis(4-sulfobutyl)-3,5-dimethylaniline, disodium salt (MADB) and 4-aminoantipyrine.
32. An at-home testing device with replaceable test strip, the device comprising:
   a light source;
   a multi-channel detector;
   a test strip insertion port adapted to receive the test strip; and
   the test strip of any one of the preceding clauses,
   wherein the light source is adapted to illuminate the optical analysis layer with visible light,
   wherein the multi-channel detector is adapted to detect reflected light from the optical analysis layer.
33. The at-home testing device of clause 32, wherein the visible light has intensity at a first wavelength and a second wavelength, wherein the first wavelength is between 400 nm and 420 nm, between 430 nm and 450 nm, between 460 nm and 480 nm, between 500 nm and 520 nm, or between 540 nm and 560 nm, wherein the second wavelength is between 573 nm and 593 nm, between 610 nm and 630 nm, or between 660 nm and 680 nm, wherein the multi-channel detector is adapted to detect light from the optical analysis layer at the first wavelength and the second wavelength.
34. The at-home testing device of clause 33, wherein the multi-channel detector measures the first wavelength and the second wavelength simultaneously.
35. A method comprising:
   a) receiving a drop of whole blood from a single fingerstick;
   b) filtering the drop of whole blood to separate a drop of plasma from other cellular blood components;
   c) exposing the drop of plasma to an enzymatic cascade detection system;
   d) measuring a rate of generation of chromophore from the enzymatic cascade detection system;
   e) applying a hemolysis correction algorithm to account for intracellular potassium; and
   f) determining a potassium concentration in the drop of whole blood using the rate of generation of chromophore and following application of the hemolysis correction algorithm.
36. The method of clause 35, the method further comprising: prior to step c), depleting the blood sample and/or the drop of plasma of sodium, ammonium, and/or calcium.
37. The method of clause 35, the method further comprising: prior to step c), depleting the blood sample and/or the drop of plasma of sodium.
38. The method of clause 35, the method further comprising: prior to step c), depleting the blood sample and/or the drop of plasma of ammonium.
39. The method of clause 35, the method further comprising: prior to step c), depleting the blood sample and/or the drop of plasma of calcium.
40. The method of clause 35, wherein the drop of whole blood has a volume of between 20 microliters and 30 microliters.
41. The method of any one of clauses 35 to the immediately preceding clause, wherein the hemolysis correction algorithm compares absorption between a first wavelength of 410 nm and a second wavelength of 620 nm.
42. The method of clause 41, wherein absorption at the first wavelength and the second wavelength are measured simultaneously.
43. The method of any one of clauses 35 to the immediately preceding clause, wherein fitting data during the measuring of step d) includes a weighted measurement using at least a portion of a standard absorbance equation and a Kubelka-Munk absorbance equation.
44. The method of the immediately preceding clause, wherein the weighted measurement is of the form Signal = X * Abs + Y * K-M where X and Y are weighting coefficients.
45. The test strip, at-home testing device, or the method of any one of the preceding clauses, wherein the enzymatic cascade detection system comprises manganese ions.
46. The test strip, at-home testing device, or the method of the immediately preceding clause, wherein a majority of divalent cations within the enzymatic cascade detection system are manganese ions.
47. The test strip, at-home testing device, or the method of the immediately preceding clause, wherein the enzymatic cascade detection system is substantially free of magnesium ions.
48. The test strip, at-home testing device, or the method of any one of the preceding clauses, wherein the enzymatic cascade detection system comprises one or more stabilizers selected from the group consisting of bovine serum albumin, sucrose, trehalose, and combinations thereof.
49. The test strip, at-home testing device, or the method of any one of the preceding clauses, wherein the enzymatic cascade detection system comprises polyethylene glycol.
50. A non-transitory computer readable medium having stored thereon instructions that, when executed by a processor, cause the processor to execute step d), e) and/or f) of the method of any one of clauses 35 to the immediately preceding clause.

The scope of the present invention is not intended to be limited to the above Description, but rather is as set forth in the following claims:

## Claims

1. A test strip comprising a layered structure comprising a top layer, a bottom layer, and active layers positioned between the top and bottom layers, the active layers including:
a filter layer adapted to separate a drop of plasma from components of a drop of whole blood from a single fingerstick;
optionally an interference mitigation composition positioned between the top layer and the bottom layer in a configuration where it contacts the whole blood and/or the drop of plasma to deplete or reduce endogenous substances therein;
an optical analysis layer comprising an enzymatic cascade detection system and adapted to receive the drop of plasma from the filter layer,
wherein the optical analysis layer is accessible to spectroscopic interrogation,
wherein the enzymatic cascade detection system includes phosphoenolpyruvate or a phosphoenolpyruvate salt, a pyruvate kinase that converts the phosphoenolpyruvate into pyruvate, and an enzyme readout system that generates a detectable chromophore from the pyruvate,
wherein the pyruvate kinase is a first stage of an enzymatic cascade,
wherein potassium concentration within the drop of plasma can be extracted from a rate of generation of the detectable chromophore from the enzymatic cascade detection system,
wherein the detectable chromophore has an absorbance peak that does not overlap with any oxyhemoglobin absorbance peak.

2. The test strip of claim 1, wherein the pyruvate kinase is from a prokaryotic species or a eukaryotic species, optionally wherein the pyruvate kinase is mammalian.

3. The test strip of any one of the preceding claims, wherein the test strip is substantially free of sodium ions, potassium ions, ammonium ions, and/or calcium ions.

4. The test strip of any one of the preceding claims, wherein the phosphoenolpyruvate salt is neither a potassium salt nor a sodium salt.

5. The test strip of any one of the preceding claims, the system comprising the interference mitigation composition,
optionally wherein the interference mitigation composition is adapted to reduce or eliminate sodium ions, calcium ions, and/or ammonium ions,
optionally wherein the interference mitigation composition comprises a coronate or a cryptate to reduce or eliminate the sodium ions and/or the calcium ions,
optionally wherein the interference mitigation composition comprises the coronate,
optionally wherein the coronate is formed from ion-crown ether complexing,
optionally wherein the interference mitigation composition comprises the cryptate,
optionally wherein the cryptate is formed from ion-cryptand complexing,
optionally wherein the interference mitigation composition comprises a crown ether, a cryptand, a chelating agent (e.g., a citrate, a gluconate, or EDTA), or a combination thereof to reduce or eliminate the calcium ions and/or the ammonium ions.

6. The test strip of any one of the preceding claims, wherein at least the optical analysis layer is a paper layer and/or wherein the active layers are paper layers.

7. The test strip of any one of the preceding claims, wherein the active layers are dried (e.g., heat dried or lyophilized).

8. The test strip of any one of the preceding claims, wherein the test strip maintains measurement accuracy for at length of time of at least one year when stored at a temperature of between 2 °C and 40 °C.

9. The test strip of any one of the preceding claims, the test strip further comprising a sample port adapted to receive the drop of whole blood.

10. The test strip of any one of the preceding claims, the test strip further comprising a foam spacer to provide physical spacing between the top layer and the bottom layer.

11. The test strip of any one of the preceding claims, wherein the enzyme readout system comprises a pyruvate oxidase, a peroxidase, and a Trinder pair,
optionally wherein the Trinder pair comprises N,N-Bis(4-sulfobutyl)-3,5-dimethylaniline, disodium salt (MADB) and 4-aminoantipyrine.

12. An at-home testing device with replaceable test strip, the device comprising:
a light source;
a multi-channel detector;
a test strip insertion port adapted to receive the test strip; and
the test strip of any one of the preceding claims,
wherein the light source is adapted to illuminate the optical analysis layer with visible light, wherein the multi-channel detector is adapted to detect reflected light from the optical analysis layer,
optionally wherein the visible light has intensity at a first wavelength and a second wavelength,
optionally wherein the first wavelength is between 400 nm and 420 nm, between 430 nm and 450 nm, between 460 nm and 480 nm, between 500 nm and 520 nm, or between 540 nm and 560 nm,
optionally wherein the second wavelength is between 573 nm and 593 nm, between 610 nm and 630 nm, or between 660 nm and 680 nm,
optionally wherein the multi-channel detector is adapted to detect light from the optical analysis layer at the first wavelength and the second wavelength, and
optionally wherein the multi-channel detector measures the first wavelength and the second wavelength simultaneously.

13. A method comprising:
a) receiving a drop of whole blood from a single fingerstick;
b) filtering the drop of whole blood to separate a drop of plasma from other cellular blood components;
c) exposing the drop of plasma to an enzymatic cascade detection system;
d) measuring a rate of generation of chromophore from the enzymatic cascade detection system;
e) applying a hemolysis correction algorithm to account for intracellular potassium; and
f) determining a potassium concentration in the drop of whole blood using the rate of generation of chromophore and following application of the hemolysis correction algorithm, optionally further comprising: prior to step c), depleting the blood sample and/or the drop of plasma of sodium, ammonium, and/or calcium,
optionally wherein the drop of whole blood has a volume of between 20 microliters and 30 microliters,
optionally wherein the hemolysis correction algorithm compares absorption between a first wavelength of 410 nm and a second wavelength of 620 nm,
optionally wherein absorption at the first wavelength and the second wavelength are measured simultaneously,
optionally wherein fitting data during the measuring of step d) includes a weighted measurement using at least a portion of a standard absorbance equation and a Kubelka-Munk absorbance equation, and
optionally wherein the weighted measurement is of the form Signal = X * Abs + Y * K-M where X and Y are weighting coefficients.

14. The test strip, at-home testing device, or the method of any one of the preceding claims, wherein the enzymatic cascade detection system comprises manganese ions,
optionally wherein a majority of divalent cations within the enzymatic cascade detection system are manganese ions,
optionally wherein the enzymatic cascade detection system is substantially free of magnesium ions.

15. The test strip, at-home testing device, or the method of any one of the preceding claims, wherein the enzymatic cascade detection system comprises one or more stabilizers selected from the group consisting of bovine serum albumin, sucrose, trehalose, and combinations thereof, and/or
wherein the enzymatic cascade detection system comprises polyethylene glycol.
